(19)

Europäisches
Patentamt

European
Patent Office

Office européen
des brevets

(11) **EP 2 857 068 A1**

(12) **EUROPEAN PATENT APPLICATION**

(43) Date of publication:
08.04.2015 Bulletin 2015/15

(51) Int Cl.:
*A61P 17/16* (2006.01) *A61K 36/484* (2006.01)

(21) Application number: **14187826.4**

(22) Date of filing: **06.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.10.2013 IT RM20130542**

(71) Applicant: **Aboca S.p.A. Societa' Agricola 52037 Sansepolcro (AR) (IT)**

(72) Inventors:
 • **Mercati, Valentino
  52037 Sansepolcro AR (IT)**
 • **Mattoli, Luisa
  52037 Sansepolcro AR (IT)**

(74) Representative: **Predazzi, Valentina et al
 Società Italiana Brevetti S.p.A.
 Piazza di Pietra, 39
 00186 Roma (IT)**

(54) **Fractions of liquorice extracts depleted of glycyrrhizic acid and uses thereof**

(57) The present invention relates to new fractions of liquorice extract in which only traces of glycyrrhizic acid, carnosic acid and derivatives thereof and resins are present, a method for the preparation of such extract, compositions comprising it, and the use of the extract and of the compositions for the protection of skin or mucous membranes.

Fig. 1a

Printed by Jouve, 75001 PARIS (FR)

(Cont. next page)

EP 2 857 068 A1

c. | clarified aqueous extract | | or | d'. | Adsorbing resin stirene DVB copolymer | → | Retentate

d. 1-ULTRAFILTRATION MWCO 2500 DALTON

Retentate > 2500 Dalton,

PERMEATE FRACTION B

ELUATE FRACTION C

Fig. 1b

**Description**

TECHNICAL FIELD OF THE INVENTION

The present invention relates to new fractions of liquorice extract in which only traces of glycyrrhizic acid are present, a method for the preparation of such fractions, compositions comprising it, and the use of the fractions and of the compositions for the protection of skin or mucous membranes.

PRIOR ART

Liquorice root is comprised of dried, decorticated and non-decorticated, whole or cut roots and stolons of *Glycyrrhiza glabra* L., a perennial or suffrutex (subshrub) plant, generally with horizontal stems below ground (stolons) high up to 1-2 m.

The spice consists of dried stolons, harvested in Autumn.

The use of liquorice root is mainly linked to therapeutic indications as adjuvant in case of gastric and duodenal ulcers or gastritis. Moreover, its action is indicated in the upper respiratory tract as expectorant in case of cough and bronchial catarrh.

Lyophilised hydroalcoholic extracts of liquorice root normally contain glycyrrhizin (a.k.a. glycyrrhizic acid or glycyrrhizinic acid) amounts greater than 7%, whose content varies according to plant provenance, variety and growth conditions.

Glycyrrhizin in liquorice root is present as salt of potassium and calcium of glycyrrhizinic acid. Glycyrrhizin is partially hydrolized *in vivo* by a glucoronidase, freeing the triterpene derivative, glycyrrhetic acid which constitutes the aglycone, which is not sweet, and two diglucuronic acid residues, which are the sugary part. Glycyrrhizin exhibits antiulcer, tumour growth inhibiting (mainly due to glycyrrhetic acid and its derivatives) mineralcorticoid (with retention of sodium and loss of potassium, leading to hypertension), antitrichomonas, antiinflammatory, antiallergic, antitoxic, antitussive (activity due to a 18-β-glycyrrhetinic acid derivative) anticonvulsive and antibacterial activities.

Glycyrrhizin moreover has side effects on the balance of mineral salts in the organism, and in case of abuse or excessive use can cause water retention, pressure increase, face and ankle swelling, headache and asthenia (mineralcorticoid effect).

The various known extraction methods are focused on improving the yield in the known active principles, or on making such components more useful for therapeutic purposes.

In the literature, it is reported how mucoadhesive materials can be useful for therapeutic purposes or to carry drugs, or as therapeutic agents as such, coating and protecting damaged tissues (for instance, gastric ulcers or lesions of the mucous membranes) (J.D. Smart 2005 Advanced drug delivery reviews 57 pp 1556-1568).

Given the medical interest for mucoadhesive substances and for those of natural origin, it is useful to develop new natural mucoadhesive compounds to be used as drug carriers or for a therapeutic/protective aim.

SUMMARY OF THE INVENTION

The present invention relates to selected fractions of liquorice extract substantially depleted in the most known pharmacologically active components represented by glycyrrhizic acid, of which only traces remain, compositions comprising such fractions and processes for obtaining them. The fractions according to the invention may be used for the protection of the mucous membranes or of the skin, to cooperate with such protection and/or also to retain desired substances on the mucous membranes thanks to the mucoadhesive and/or barrier effect abilities of the selected fractions even in the absence, therefore, of glycyrrhizic acid. The fractions of the invention therefore provide a new source of material of plant origin useful for the protection of the mucous membranes and of the skin and/or also as excipient capable of retaining for a longer time desired substances on the mucous membranes, wherein the pharmacological effects attributable to the components known as active principles, are substantially reduced or even eliminated.

Said condition is particularly desirable when materials of natural or plant origin are to be used for actions of a mainly mechanical type (i.e., not for pharmacological actions having, for instance, a direct effect on the activation of receptorial, immune pathways or metabolic processes). The use of substances having a mainly mechanical effect, such as, e.g., substances having a barrier or mucoadhesive effect, is desirable, for instance, to cooperate with a pharmacological therapy. By minimizing the presence of substances with pharmacological activity in the material of plant or natural origin as in present invention, it is obtained a material with a mainly mechanical effect that can be used in combination with drugs, minimizing possible undesired interactions with drugs, and at the same time exploiting the typical feature of substances of plant origin, of having a vast number of components capable of exerting a protective effect besides the pharmacologically active ones commonly used.

The use of fractions of plant or natural extracts depleted in the most known pharmacologically active principle such as glycyrrhizic acid, and enriched in the remaining substances, entails the dual advantage of providing a material with a mainly mechanical protective action (mucous membrane protection or skin protection) and of enabling a concrete and useful use of materials normally discarded in extraction processes of plant or natural active principles.

The present invention therefore relates to fractions of liquorice extracts depleted in glycyrrhizic acid, with a glycyrrhizic acid concentration, expressed as weight percent of the lyophilised fraction, of ≤1% or even of ≤0.8% or even of ≤0.4% or even of ≤0.05%; their use as in the protection of the skin or mucous membranes and/or as carriers with a high mucoadhesive ability; compositions comprising such fractions; their use in the protection of the skin or mucous membranes and/or for the carrying of desired active principles on the mucous membranes and processes for the preparation of such fractions and of such compositions.

The fractions of the invention are characterized by mucoadhesive and barrier effect properties greater than those of conventional deglycyrrhizinated liquorice extracts (glycyrrhizic acid about 3%), of the conventional hydroalcoholic liquorice extracts and of the reference liquorice extract used in the present description, represented by the lyophilisate of the liquorice root and/or taproot extract in 30% alcohol.

The invention also relates to a process for the preparation of fractions of liquorice extract as defined above, comprising the following step:

> a. preparing a hydroalcoholic extract of liquorice root and/or taproot and subjecting said extract to centrifugation and/or decantation, obtaining a precipitated fraction A, and a clarified alcoholic extract, and said precipitated fraction A is collected characterised by a glycyrrhizic acid concentration, expressed as weight percent, of ≤1 or even of ≤0.8.

The process can further comprise additional steps:

> b. obtaining from the clarified alcoholic extract prepared in a. a concentrated aqueous extract,
> c. decanting and/or centrifuging and filtering said aqueous extract, collecting a water-soluble fraction:
> d. subjecting the water-soluble fraction obtained in c. to membrane ultrafiltration and collecting a fraction B, corresponding to the permeate obtained from said ultrafiltration, characterised by a glycyrrhizic acid concentration, expressed as weight percent, of ≤0.05% of even of ≤0.03.

Or, the process can comprise further steps:

> b. obtaining from the clarified alcoholic extract prepared in a. a concentrated aqueous extract,
> c. decanting and/or centrifuging and filtering said aqueous extract, collecting a water-soluble fraction:
> d'. subjecting the water-soluble fraction obtained in c. to a step on adsorbing resin collecting a fraction C, corresponding to the eluate obtained by said step on resin, characterised by a glycyrrhizic acid concentration, expressed as weight percent, of ≤0.4%.

The invention also relates to the preparation of said fractions A, B and/or C, and the preparation of mixtures of said fractions A and/or B and/or C with one or more natural extracts and/or active principles deriving from sources different from liquorice, such mixtures, as well as the preparation of pharmaceutical compositions or of medical foods containing one or more of said fractions in combination with other natural substances and/or active principles deriving from sources different from liquorice and the use of such fractions alone or mixed between them or with other components, for the protection of the mucous membranes and of the skin or as carriers capable of retaining desired substances (deriving from sources different from liquorice) on the mucous membranes by virtue of their effective mucoadhesive properties.

As to medical foods, there could be used the fractions rich in active principles, obtained as waste of the process for enriching foods with said active principles.

The invention therefore relates to fractions of liquorice extracts and uses thereof in the protection of mucous membranes or of the skin thanks to their mechanical features of mucoadhesion and/or barrier effect, or also as carriers with good mucoadhesive properties substantially free from the effects attributable to liquorice active principles commonly used, i.e. glycyrrhizic acid.

The percentage values are referred to the weight of the fractions object of the invention after drying treatment by lyophilisation, therefore by "expressed as weight percent of" it is meant "expressed as weight percent of the lyophilised fraction" or "expressed as weight percent of the lyophilised extract".

DETAILED DESCRIPTION OF THE FIGURES

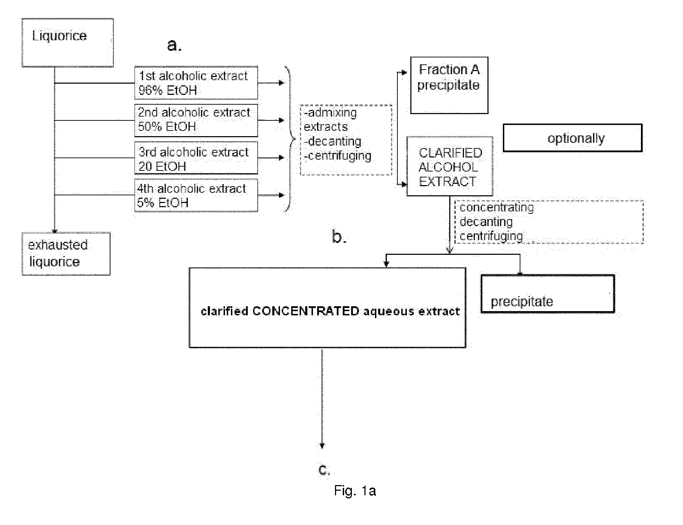

Figure 1a shows a block diagram of an embodiment of the process of the invention, comprising steps a and b

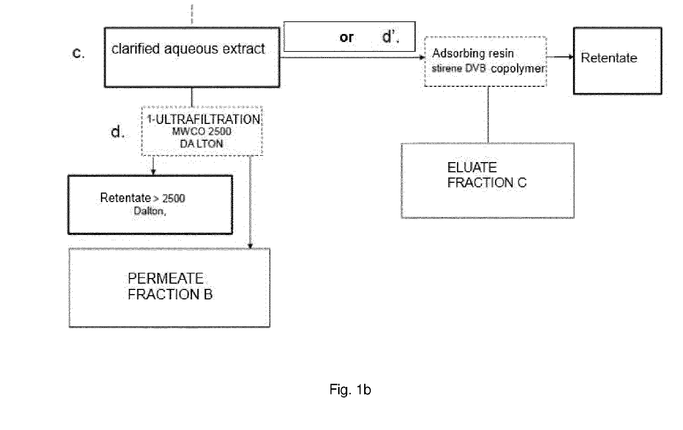

Figure 1b shows a block diagram from step c on.

SHORT GLOSSARY

NMWCO=Nominal Molecular Weight Cut-off
PERMEATE: extract that has passed through the semi-permeable ultrafiltration membrane
RETENTATE: extract that did not pass through the ultrafiltration membrane or material adsorbed on resin.
ELUATE: material not adsorbed on resin following adsorption passages thereon.
ULTRAFILTRATION: filtration technique on semi-permeable membrane characterised by pores having sizes of
100000 daltons (0.1 $\mu$m) to 500 daltons (about 0.005 $\mu$m)
LIQUORICE PARTS USED: roots and/or taproot
By "NOT GREATER THAN", with respect to the amounts of active principles in the extract, in the present description
it is meant $\leq$.
GLYCYRRHIZIC ACID: in the present description, the glycyrrhizic acid concentrations indicated are quantitatively
determined as weight percent of the sample lyophilised by HPLC chromatographic method.
The TOTAL REFERENCE EXTRACT or reference extract in the present invention is represented by the lyophilisate
of liquorice root and/or taproot extract in 30% ethanol.

DETAILED DESCRIPTION OF THE INVENTION

As indicated above, in the present description there are provided fractions of liquorice extract depleted in
glycyrrhizic acid and/or with a glycyrrhizic acid concentration of $\leq$1% or also of $\leq$0.8% or also of $\leq$0.4% or also of $\leq$0.05%
by weight, having good mucoadhesive and/or barrier effect abilities on the mucous membranes.
By applying the fractioning processes described below, it was possible to obtain fractions of liquorice extract
depleted in glycyrrhizic acid and concomitantly enriched in the remaining substances, which exhibited an effective
protective action on the skin and on the mucous membranes as evident from the mucoadhesivity assays performed on
cells and/or from the barrier assays reported in the experimental section below.
With respect to liquorice fluid, soft, lyophilised or dry extracts commonly used, which comprise an amount of
glycyrrhizic acid equal to about 7% by weight, the fractions of the present invention comprise amounts of glycyrrhizic
acid of $\leq$1% or even of $\leq$0.8% or even of $\leq$0.4% or even of $\leq$0.05% by weight.
The fractions according to the present description exhibit surprising mucoadhesive and/or barrier effect activities.
Such activities are greater than those of the reference extract, represented by the lyophilisate of the liquorice
root and/or taproot extract in 30% alcohol, which is a conventional liquorice extract, and are also better than those of
the known deglycyrrhizinated liquorice extract (glycyrrhizic acid about 3%) having, moreover, a glycyrrhizic acid percent
of <1%.
Evidently, such values of glycyrrhizic acid correspond in practice to traces of glycyrrhizic acid. Such values
cannot be held accountable for the mucoadhesive and barrier properties exhibited by the fractions of the invention and
cannot induce the pharmacological activities for which liquorice or liquorice extracts are commonly used.
The selected fractions according to the present description, substantially depleted in active principles like
glycyrrhizic acid, preserve however good mucoadhesive and/or barrier effect properties as can be seen in Table 1 below.
The table below shows the mucoadhesive (by adhesion method with mucin) and barrier effect properties of the
fractions with respect to a total lyophilised liquorice extract used as control.
The assessment of mucoadhesive properties was carried out by adhesion assay with mucin on an inclined
plane described in detail in the experimental section below. The barrier effect was instead measured by cell marker
release inhibition assay in response to an inflammatory or irritating agent as described in detail in the experimental
section below.

**Table 1**

| ANALYZED SAMPLE | Barrier effect assay NET BARRIER EFFECT: % of IL-6 inhibition of the B.A. -% of IL-6 inhibition of the IC | Adhesion assay with mucin on an inclined plane -% | Glycyrrhizic acid concentration expressed as % by weight | Water-soluble macromolecules with a molecular weight of ≥25000 Da |
|---|---|---|---|---|
| Reference liquorice extract, lyophilised | 20 | 14 | 7.5 | 38.84 |
| liquorice, fraction A, lyophilised | 38 | 22 | 0.77 | 17.53 |
| liquorice, fraction C, resin eluted fraction, lyophilised | 54 | >80 | 0.03 | 19.84 |
| Liquorice, fraction B, gh permeate <2500 da, lyophilised | 45 | 41 | 0.34 | 14.78 |

The depletion in the most known active principles of liquorice, represented by glycyrrhizic acid, is directly measurable by the measurement of weight percent (% by weight) of glycyrrhizic acid in HPLC.

The table highlights how the fractions of the invention are not mere dilutions of the reference extract, given the evident increase of ratio between the glycyrrhizic acid percentage and the water-soluble macromolecules with a molecular weight of >25000 Da.

The measurement of glycyrrhizic acid as reported in the present description is obtainable with standard processes by HPLC chromatographic method.

Said method of analysis provides an extraction of the sample under examination, with an ammoniacal aqueous solution containing 25% ammonia, by ultrasounds at a temperature of 35°C ±3°C; and HPLC analysis by c18 reverse-phase column and UV detector at a wavelength of 254 nm.

The reference extract has a glycyrrhizic acid concentration equal to about 7.3%, a composition in water-soluble macromolecules with a molecular weight of <25000 Da equal to about 38.8%, a net barrier effect calculated with the process described below equal to about 20% of inhibition of IL-6, and a mucoadhesion, expressed as adhesion percentage on an inclined plane, calculated with the process described below, equal to about 22%.

This fraction therefore exhibits a ratio between glycyrrhizic acid and water-soluble macromolecules with a molecular weight of ≥ 25000 Da equal to about 1:5.

The barrier effect is evaluated according to the process described below and the mucoadhesive properties are evaluated by a cell adhesion assay, it also described below. Evidently, such properties can also be evaluated with further techniques known to a person skilled in the art.

In particular, fraction A of the invention has a glycyrrhizic acid concentration of ≤0.8% by weight, markedly reduced with respect to the starting extract, resulting in a depletion in glycyrrhizic acid greater than 89% with respect to the starting extract. This fraction has a composition in water-soluble macromolecules with a molecular weight of >25000 Da, equal to about 17.5%, a net barrier effect calculated with the process described below equal to about 38% of inhibition of IL-6, and a mucoadhesion, expressed as adhesion percentage on an inclined plane calculated by the process described below, equal to about 22%. Fraction A is therefore depleted of about 88% in the active principle glycyrrhizic acid with respect to the reference liquorice extract, represented by the lyophilisate of the extract in 30% alcohol, and exhibits a barrier effect greater than the reference extract.

This fraction therefore has a ratio between glycyrrhizic acid and water-soluble macromolecules with a molecular weight of 25000 Da equal to about 1:25.

Fraction B of the invention has a glycyrrhizic acid concentration of ≤0.05% by weight markedly reduced with respect to the starting extract, resulting in a depletion in glycyrrhizic acid greater than 99% with respect to the starting extract. This fraction has a composition in soluble macromolecules with a molecular weight of >25000 Da equal to about 14.8%, a net barrier effect calculated with the process described below equal to about 45% of inhibition of IL-6, and a mucoadhesion expressed as adhesion percentage on an inclined plane by the process described below equal to about 41%. Fraction B is then depleted of about 99% in the active principle glycyrrhizic acid with respect to the reference liquorice extract, represented by the extract lyophilisate in 30% alcohol and has a barrier effect equal to 2.5 times that of the reference extract and also a mucoadhesion ability it also equal to 2.5 times that of the reference extract.

This fraction therefore has a ratio between glycyrrhizic acid and water-soluble macromolecules with a molecular weight of 25000 Da equal to about 1:509, therefore ≤1:500.

Fraction C of the invention has a glycyrrhizic acid concentration of ≤0.4% by weight, markedly reduced with respect to the starting extract, resulting in a depletion in glycyrrhizic acid greater than 94% with respect to the starting extract. This fraction has a composition in soluble macromolecules with a molecular weight of >25000 Da equal to about 19.8%, a net barrier effect calculated by the process described below equal to about 54% of inhibition of IL-6, and a mucoadhesion expressed as adhesion percentage on an inclined plane, calculated by the process described below, greater than about 80%. Therefore, fraction C is depleted of about 94% in the active principle glycyrrhizic acid with respect to the reference liquorice extract, represented by the extract lyophilisate in 30% alcohol, and has a barrier effect equal to almost three times that of the reference extract and a mucoadhesion ability at least four times that of the reference extract.

This fraction therefore has a ratio between glycyrrhizic acid and water-soluble macromolecules with a molecular weight of 25000 Da equal to about 1:58, therefore ≤1:50.

All fractions therefore have a ratio between glycyrrhizic acid and water-soluble macromolecules with a molecular weight of 25000 Da of ≤1:20.

According to the above-described features of fractions A, B and C, the fractions of the invention will be depleted in glycyrrhizic acid of at least the 89% up to over 99% by weight, e.g. at least 89, 90, 91, 92, 93, 94, 95, 96, 97, 99, 99% and exhibit a barrier effect equal to at least twice with respect to the reference liquorice extract, represented by the extract lyophilisate in 30% alcohol which has not undergone fractioning.

The Inventors of the present invention have selected the above-mentioned fractions with respect to other fractions obtainable with the process as described in the present document, as they were the only ones exhibiting sufficient reductions in glycyrrhizic acid and improving (despite at times having similar compositions) in the mucoadhesive and/or barrier effect abilities with respect to a reference extract represented by the liquorice extract lyophilisate in 30% alcohol.

Many of the fractions obtained, in fact, besides exhibiting an enrichment in liquorice active principles, (marked pharmacological effect), in some cases exhibited a non-interesting barrier or mucoadhesive effect.

Table 2 below reports data related to the various fractions analyzed and shows how the barrier/mucoadhesive effect cannot be directly related with the concentration of glycyrrhizic acid and/of water-soluble macromolecules with a molecular weight of >25000 Da.

Fraction A corresponds to the precipitate obtained with the process of the invention, from mixing the first extract in 96% ethanol with the successive extract in 5-15% ethanol, in a 50:50 ratio or in alternative percentage so as to keep the alcoholic grade of the resulting mixture comprised between 40° and 60°.

Fraction D corresponds to the water-insoluble precipitate, obtained by alcohol evaporation of the alcoholic extract deprived of fraction A.

Fraction E corresponds to the hydrosoluble fraction obtained after having removed fractions A and D.

Fraction F corresponds to the adsorbed fraction obtained by subjecting the water-soluble fraction obtained in c. to a step on high-porosity adsorbing styrene and DVB copolymer resin.

Fraction C corresponds to the NOT adsorbed fraction obtained with the process of the invention, by subjecting the water-soluble fraction obtained in c. to a step on high-porosity adsorbing styrene and DVB copolymer resin.

Fraction G corresponds to the retentate fraction obtained by subjecting the water-soluble fraction obtained in c. to a step on filter with a cutoff of 2500 Da.

Fraction B corresponds to the eluted fraction obtained, with the process of the invention, by subjecting the water-soluble fraction obtained in c. to a step on filter with a cutoff of 2500 Da.

Table 2

| Sample description | Glycyrrhizic acid in HPLC (%) | Water-soluble macromolecules greater than 25000Da (%) | Adhesion with mucin (method on an inclined plane) - % | BARRIER EFFECT % of inhibition of IL-6 release (20mg/ml concentration) |
|---|---|---|---|---|
| Reference liquorice extract, lyophilised | 7.311 | 38.84 | 14.0 | 20 |
| Liquorice first fraction, 1st precipitate, lyophilised FRACTION A | 0.766 | 17.53 | 22.0 | 38 |
| Liquorice second fraction D, 2nd precipitate, lyophilised | 2.445 | 2.16 | 23.0 | 61 |
| Liquorice third polar fraction E, lyophilised | 7.732 | 25.04 (*) | 111.0 | 34 |
| Liquorice fraction F, adsorbed by resin, lyophilised | 10.146 | 45.18 (*) | 10.0 | 0 |
| Liquorice fraction NOT adsorbed by resin, lyophilised FRACTION C | 0.337 | 19.84 | 104.0 | 54 |
| Liquorice fraction G retentate GH >2500 da, lyophilised | 12.929 | 16.10 | 66.0 | 20 |
| Liquorice fraction permeate, GH <2500 da, lyophilised FRACTION B | 0.029 | 14.78 | 41.0 | 45 |

Therefore, evidently it is difficult to foresee which fractions of a plant extract be capable of exhibiting a barrier and/or mucoadhesion effect and how the fractions of the invention have mucoadhesive activities definitely greater than those of the reference extract and those of fraction E that exhibits the features, in terms of glycyrrhizic acid percentages, more similar to those of deglycyrrhizinated liquorice described in the literature.

Moreover, all selected fractions exhibit a barrier effect greater than that of the reference extract.

In the present invention, the mucoadhesion percentage could be measured according to any method known to the technician in the field. In particular, the mucoadhesion percentage can be measured according to a simple mucoadhesion assay on an inclined plane that is explained in a detailed manner in the experimental section and which, in short, consists in measuring the mucoadhesive ability of a sample by measuring its ability to bind to mucin disposed on an inclined plane, allowing the moving away by sliding of the sample lacking mucoadhesive ability.

The method provides the use of an inclined plane which acts as support for the biological substrate, consisting of a mucin film set up by depositing, on a plexiglas plane kept horizontal, a suspension of gastric mucin (e.g., porcine) at a certain concentration in a buffer with physiological pH.

The plane is dried to obtain a film of known surface area.

An amount of known weight of the sample to be analyzed is prepared and deposited on the measurement plane with a multichannel pipette at constant time and rate for some seconds.

The total amount is measured at each assay.

The sample which does not stop on the plane is collected and measured at the end of the run. The amount of fallen sample is measured by recording the weight variation as a function of time. As control, measurements in the absence of the mucin film on the inclined plane are also performed (measurements indicated as blank) with the same amounts of blank and under the same assaying conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

% difference = (adhered mucin % - adhered blank %)/ adhered % of the blank)

Where:

- adhered mucin % = sample percentage remained adhered to the mucin film at the end of the measurement
- adhered blank % = sample percentage at the inclined plane at the end of the blank measurements.

The barrier effect of a compound on mucous membranes or skin may be measured in any way available to the technician in the field. For instance, the barrier effect may be measured by cell marker release inhibition assay in response to an inflammatory or irritating agent as described in detail in the experimental section below.

In short, however, an *in vitro* method for the assessment of the barrier effect of fractions of extract of interest on a cell culture can be summarized with the following steps:

a) preparing a cell culture;
b) placing the sample of interest (e.g., one of the fractions according to the invention) on a semi-permeable membrane separating it from said culture, and, subsequently or concomitantly, adding to said sample a substance (e.g., an inflammatory agent such as bacterial lipopolysaccharide LPS or an interferon; an irritating or allergenic agent like ovoalbumin, dinitrochlorobenzene, oxazolone, eugenol, penicillin G, nickel sulphate) that, when in contact with said culture, be able to induce release of a marker (e.g., histamine or $\beta$-glucuronidase or a cytokine like IL-8, IL-6 , IL1$\alpha$, TNF$\alpha$, TNF$\beta$) from said culture;
c) detecting in one or more successive time instants the concentration of said marker in the experiment prepared in a) and b) and comparing the measurements obtained relative to one or more reference values, wherein the lack of increase over time of said marker in step c. denotes a barrier effect of the sample.

In particular, step c) can comprise:

detecting in one or more successive time instants the concentration of said marker in the experiment prepared in a) and b) and in a parallel control experiment in which the extract fraction of the invention is not added on the permeable membrane in b) and comparing the measurements obtained from the two experiments in the same time instants.

Step c) could also comprise:

detecting in one or more successive time instants the concentration of said marker in the experiment prepared in steps a) and b) relative to the amount of said marker measured in the same experiment before step b).

In one embodiment the method can further comprise a further step of preparing an internal control in which cultured cells are pre-treated with the substance adapted to induce marker release and the extract fraction of the invention is placed on the semi-permeable membrane in the absence of said substance, which could be added later to the fraction under examination in case said substance were to settle before said fraction.

According to one embodiment, the barrier effect is measured by IL-6 inhibition assay in response to an agent such as LPS.

The values provided in the present invention are all referable to the above-indicated methods.

The fractions of the invention, represented by fractions A, B and/or C or by mixtures thereof, are therefore useful for their mucoadhesion and/or barrier effect properties in all those cases in which a protection of skin or mucous membranes is needed or desirable. This may occur in those cases wherein a drug which attacks the mucous membranes or the skin has to be administered, and therefore the fractions have the purpose of preventing or anyhow limiting the damaging action of the drug. It may also occur in those cases in which the protection of a partially compromised skin or mucous membrane is preferable or advisable so as to enable a better and quicker healing thereof, defending it from further aggressions, or in those cases in which an individual has a chronic disorder in which skin or mucous membranes undergo irritations or alterations whereby a barrier effect can prevent or limit damages on the skin or the mucous membrane, or again in those cases in which it is desired to carry particular substances in a preferential way and with a prolonged duration on the mucous membranes.

By "protection of the mucous membranes" it is meant a mechanical protection, given by the mucoadhesive or barrier abilities of the fractions of the invention or of mixtures thereof, which could be associated with a pharmacological protection related to active principles mixed with the fractions of the invention. Just as a bandage physically protects a wound and prevents further worsenings of the same, thereby facilitating its healing, the barrier effect of the fractions of the invention protects skin and mucous membranes from aggression of principles that may cause pathologies and irritations and that can slow down the healing of compromised skin or mucous membranes. In the present description said mucous membranes can be the oral mucosa, gastric mucosa, intestinal mucosa, the nasal mucosa, the vaginal mucosa, the uterine mucosa, the rectal mucosa.

Fractions A, B and C could be used alone or mixed with each other and could be optionally further mixed (alone or in combination) with extracts of other plants and/or natural active principles of other origin (i.e., not extracted from liquorice) as active principles of other plants or of other natural products in order to boost the mechanical activity, as defined above, of the resulting mixtures.

According to the invention, the fractions could be obtained by the process described hereinafter.

Process for the preparation of fractions of liquorice extract depleted in glycyrrhizic acid comprising the following step:

a. preparing a hydroalcoholic extract of liquorice root and/or taproot and subjecting said extract to centrifugation and/or decantation, obtaining a precipitated fraction A, and a clarified alcoholic extract, and said precipitated fraction A is collected. This fraction is characterised by a glycyrrhizic acid concentration, expressed as weight percent of the lyophilised fraction, of ≤0.8%.

The process can comprise further steps:

b. obtaining from the clarified alcoholic extract prepared in a. a concentrated aqueous extract,
c. decanting and/or centrifuging and filtering said aqueous extract, collecting a water-soluble fraction;
d. subjecting the water-soluble fraction obtained in c. to membrane ultrafiltration and collecting a fraction B, corresponding to the permeate obtained from said ultrafiltration, characterised by a glycyrrhizic acid concentration expressed as weight percent of the lyophilised fraction of ≤0.05%.

Or, the process may comprise further steps:

b. obtaining from the clarified alcoholic extract prepared in a. a concentrated aqueous extract,
c. decanting and/or centrifuging and filtering said aqueous extract, collecting a water-soluble fraction:
d'. subjecting the water-soluble fraction obtained in c. to a step on adsorbing resin collecting a fraction C, corresponding to the eluate obtained by said step on resin, characterised by a glycyrrhizic acid concentration, expressed as weight percent of the lyophilised fraction, of ≤0.4%.

The fraction A obtainable by the above-described step a. exhibits quite interesting features reported in Table 1 above, i.e.:

glycyrrhizic acid concentration of ≤ 0.8%, barrier effect measured with the assay as described below

$$BE = \%\ \text{reduction in release of cytokine IL-6} = 100 - [\ (pg/\mu L\ \text{cytokines released from sample} / pg/\mu L\ \text{cytokines released from C} +) \times 100]$$

equal to about 38% of inhibition of IL-6, mucoadhesion percentage by adhesion assay reported below equal to about 19%.

The fraction B obtainable by the above-described step d. exhibits very interesting features reported in Table 1 above, i.e.:

glycyrrhizic acid concentration ≤ 0.05%, barrier effect measured with the assay as described below

$$BE = \% \text{ reduction in release of cytokine IL-6} = 100 - [ \text{ (pg/µL cytokines released from sample / pg/µL cytokines released from C +) x 100]}$$

equal to about 45% of inhibition of IL-6, mucoadhesion percentage by adhesion assay reported below equal to about 39%.

The fraction C obtainable by the above-described step d' exhibits very interesting features reported in Table 1 above, i.e.:

glycyrrhizic acid concentration ≤ 0.4%, barrier effect measured with the assay as described below

$$BE = \% \text{ reduction in release of cytokine IL-6} = 100 - [ \text{ (pg/µL cytokines released from sample / pg/µL cytokines released from C +) x 100]}$$

equal to about 54% of inhibition of IL-6 .

The process according to the invention could then be used to produce one or more of the fractions of interest described herein, A, B or C.

The process described herein could be carried out starting from liquorice root and/or taproot, both fresh and dried, or alternatively from fluid extracts (like, e.g., hydroalcoholic extracts) or dry liquorice extracts.

The dry extracts could be resuspended/resolubilised by means of hydroalcoholic solutions, e.g. by using a solid/solvent ratio of 1:5, or 1:7 or 1:8 or 1:10, putting under stirring the mass for a time ranging from 4 to 8 hours.

Step a. of the process:

a. preparing a hydroalcoholic extract of liquorice root and/or taproot and subjecting said extract to centrifugation and/or decantation, obtaining a clarified alcoholic extract; according to the invention, the above-described process can be carried out by performing at step a. at least two, at least three, or at least four steps in ethanol at decreasing concentrations, wherein the decreasing concentrations of ethanol are from about 96% ethanol to about 0% ethanol.

For instance, step a. of the process can be carried out by performing a step in about 96% ethanol, and a step in about 5%-15% ethanol.

Or, step a. of the process could be carried out by performing a step in about 96% ethanol, a step in about 50% ethanol and a step in about 20% ethanol.

Or, step a. of the process could be carried out by performing a step in about 96% ethanol, a step in about 50% ethanol and/or a step in about 20% ethanol and a step in about 5% ethanol. The extracts obtained as described above are gathered to form a hydroalcoholic extract. These can be gathered, e.g., in equal mutual ratios, such as 50:50 in case of two steps in ethanol, or such as 33.3:33.3:33.3 in case of three steps in ethanol or 25:25:25:25 in case of four steps in ethanol. Evidently, the extracts obtained by performing the various steps in ethanol described above could be gathered into different ratios according to the general knowledge of the technician in the field.

In any case, the alcoholic grade of the resulting mixture will be of from 40° to 60°, preferably from 45° to 55° alcoholic grades.

This particular alcoholic content enables to obtain fraction A as described above, which could be lyophilised and used according to the invention.

As already indicated above, the clarified alcoholic extract (or hydroalcoholic extract) obtained in step a. could be used in successive steps b. c. and d. or b. c. and d'.

Initially, the hydroalcoholic extract obtained in a. will then be used to prepare a concentrated aqueous extract in step b.

Said concentrated aqueous extract could be prepared by concentrating the hydroalcoholic extract obtained in a. by alcohol evaporation, then it is decanted and/or centrifuged and/or filtered and the resulting concentrated aqueous phase is collected. For instance, in case of static and/or centrifugal decantation, the aqueous supernatant is collected whereas in case of filtration the filtrate is collected. Alternatively, alcohol evaporation could be performed after the centrifugation and/or decantation and/or filtration steps.

Decantation could be performed, e.g., 1 to 72 hours, and could be followed or replaced by one or more centrifugations to about 3000-4000 (e.g., about 3500) rpm for a period comprised between 1 and 10 minutes, e.g. about 5 minutes. For the filtration suitable filters known to a technician in the field could be used, like, e.g. panel filters having a cutoff of about 25 micrometers. In some cases, further consecutive filtrations of the extract could be performed on filters having gradually decreasing cutoffs, from 200 micrometers down to 0.1 micrometers.

The supernatant or the filtrate obtained after this step or steps is then collected and subjected, in case this has not already been done before, to alcohol evaporation by standard techniques, like e.g. by use of thin film distillation system, or a batch concentration system according to standard protocols commonly used by the technician in the field.

The result of this evaporation is a concentrated aqueous extract that, in step c. of the process, is in turn subjected to decantation and/or filtration.

As already mentioned, this evaporation could be performed also before the step of decantation and/or centrifugation and/or filtration.

The concentrated aqueous extract (which could be indicated in the text or in the drawings as concentrated aqueous solution) can be subjected to decantation for a period comprised between 1 and 72 hours, and the supernatant is then collected, thereby obtaining a clarified or clear solution.

Decantation could be followed or replaced by one or more centrifugations to about 3000-4000 (e.g., about 3500) rpm for a period comprised between 1 and 10 minutes, e.g. about 5 minutes.

Alternatively, the concentrated aqueous extract is filtered, thereby obtaining a clarified solution. In some cases, further consecutive filtrations of the extract could be performed on filters having gradually decreasing cutoffs, starting from 200 micrometers until arriving to 0.1 micrometers. the filtration could be performed, for instance, on a panel filter having a cutoff of 25 micrometers.

In a further embodiment the concentrated aqueous extract could be decanted as described above and the supernatant obtained could then be filtered as described above.

The supernatant or the filtrate obtained as described above are collected and subjected to a further step of depletion in glycyrrhizic acid by ultrafiltration or passage on adsorbing resin.

The method of the invention therefore comprises a further step, described below as d or d', of depletion of undesired substances from the supernatant or the filtrate obtained in step c. described above before collection of the desired extract, wherein said supernatant or filtrate is subjected to one or more further steps of depletion in glycyrrhizic acid.

This further step will enable to obtain further fractions of liquorice extract comprising glycyrrhizic acid at a concentration expressed as weight percent of ≤0.05% in case step d. is performed, or comprising glycyrrhizic acid at a concentration expressed as weight percent of ≤0.4% in case step d' is performed.

This step could be carried out by ultrafiltration of the supernatant or filtrate obtained in c. (step d.) or by passage on adsorbing resin of the supernatant or filtrate obtained in c. or even of the retentate obtained after ultrafiltration (step d'.).

In the first case the supernatant or filtrate obtained in c. is subjected to one or more ultrafiltration steps and the permeate (fraction B) is recovered, wherein said permeate comprises glycyrrhizic acid at a concentration weight percent of ≤0.05%.

In this further step, the aqueous extract (result of the filtration or supernatant of the decantation in c.) is subjected to one or more steps of ultrafiltration on membrane with a molecular weight cutoff (NMCWO) of from 500 to 50000 daltons, e.g. of 10,000 daltons, 5,000 daltons, 2,500 daltons, 1,000 daltons. The permeate extract thus obtained is substantially free from glycyrrhizic acid and can be used as is or subjected to a lyophilisation process to provide a lyophilised extract useful for formulations having therapeutic employ for internal and/or external use.

According to the invention, therefore, said one or more ultrafiltration steps could be performed on a membrane having a cutoff of about 500-50,000 daltons, e.g. about 10,000 daltons, 5,000 daltons, 2,500 daltons, 1,000 daltons.

In a preferred embodiment, a membrane having a cutoff of about 2500 Daltons will be used.

Curiously, even with this type of cutoff the glycyrrhizic acid, which by nominal size should have passed through the membrane, is instead abundantly retained, enabling to obtain a permeate (FRACTION B) with excellent mucoadhesive and barrier effect properties and with a glycyrrhizic acid concentration lower than 0.03% by weight.

The present invention therefore relates to a process for the preparation of fractions of liquorice extract depleted in glycyrrhizic acid, comprising the following steps:

a. preparing a hydroalcoholic extract from liquorice root and/or taproot and subjecting said extract to centrifugation and/or decantation, obtaining a precipitated fraction A and a clarified alcoholic extract, and collecting said precipitated fraction,
b. obtaining from the clarified alcoholic extract prepared in a. a concentrated aqueous extract,
c. decanting and/or filtering said aqueous extract, collecting a water-soluble fraction (supernatant or filtrate),
d. subjecting the water-soluble fraction obtained in c. (supernatant or filtrate obtained in c) to one or more ultrafiltration steps and the permeate (fraction B) is recovered; wherein said permeate comprises glycyrrhizic acid at a concentration expressed as weight percent of ≤0.05%.

According to a further alternative embodiment the method of the invention could comprise, alternatively or in addition to step d. described above, a step

defined herein as step d'. wherein the filtrate or the supernatant obtained in c. or the permeate obtained after the above-described ultrafiltration, is subjected to one or more passages on high-porosity adsorbing resin and the eluate is collected, wherein said eluate (fraction C) thus obtained comprises glycyrrhizic acid at a concentration expressed as weight percent of ≤0.4%.

In particular, the resin will be a resin able to adsorb aromatic and/or nonpolar substances, like, e.g., a hydrophobic high-porosity adsorbing resin. This type of resin is constituted by microspheres of a diameter of 0.2 mm - 0.8 mm, with an uniformity coefficient equal to a maximum of 1.5, obtained by polymerization of Styrene and DVB without active groups, characterised by a highly porous physical structure having the parameter related to the pore volume equal to about 1.3 ml/g, enabling the adsorption and the selective elution of organic substances, preferably of aromatic nature. A non-binding example of resin useful for the carrying out of the process consists, e.g., of amberlite XAD-2, serdolit® PAD-II, ADS TQ 318 or resins similar thereto.

The treatment performed in this embodiment is a bland and neutral treatment. Surprisingly, the resin, which is a resin type ideal to retain aromatic substances, also retains glycyrrhizic acid which is a triterpenic saponin.

Therefore, the present invention also relates to a process for the preparation of fractions of liquorice extract depleted in glycyrrhizic acid, comprising the following steps:

a. preparing a hydroalcoholic extract from liquorice root and/or taproot and subjecting said extract to centrifugation and/or decantation, obtaining a precipitated fraction A and a clarified alcoholic extract, and collecting said precipitated fraction,

b. obtaining from the clarified alcoholic extract prepared in a. a concentrated aqueous extract

c. decanting and/or filtering said aqueous extract, collecting a water-soluble fraction (the supernatant or the filtrate);

d'. subjecting said water-soluble fraction obtained in c. (the filtrate or the supernatant obtained in c.) or the permeate obtained from the ultrafiltration in d. to one or more steps on a hydrophobic adsorbing resin and the eluate (fraction C) is collected.

wherein said eluate thus obtained comprises glycyrrhizic acid at a concentration expressed as weight percent of ≤0.4%.

According to the present invention the step in d' can be carried out on a column with resins as described above, able to adsorb aromatic or highly nonpolar substances letting elute non-aromatic polar substances. The resin-adsorbed material, if desired, could be desorbed with a suitable solvent, like e.g. ethanol or alcoholic solvents, for other uses.

Suitable chromatography resins may be, for instance, high-porosity adsorbing resins of styrene divinylbenzene copolymer like, e.g., amberlite XAD-2, serdolit PAD-II or the like. The step on resins having adsorbing features of the above-described type enables therefore to obtain a fraction depleted in glycyrrhizic acid (≤0.4%).

The passage on resin could be carried out alternatively or in addition to the above-described ultrafiltration step; each of said steps could be repeated more than once and the extract obtained could then be concentrated if desired.

The extract obtained with the above-indicated method will contain only traces also of the other substances having a phenolic structure present in the initial product, besides glycyrrhizic acid.

The further step d and/or d' therefore enables a further elimination of glycyrrhizic acid.

Therefore, the process according to the present invention enables to obtain fractions of liquorice extract as described above, depleted in glycyrrhizic acid and surprisingly rich in substances having a protective action.

Moreover, the above-described process can also eliminate resins by means of the reagents and steps used, as evident to the technician in the field.

Hence, in case of fractions B and C, the fractions of the invention will also be substantially free from resins, that will be found in the discarded fractions.

The present invention also relates to a composition comprising one or more fractions of a liquorice extract useful in the protection of the skin or of the mucous membranes wherein said fraction comprises a concentration of glycyrrhizic acid not greater than that of said fractions, in particular, said composition comprises, as glycyrrhizic acid, only the glycyrrhizic acid present in said fractions.

Said protection, of course with reference both to the fractions (A, B, C, AB, AC, BC) per se and to the composition, could be a preventive protection or a protection of partially compromised skin or mucous membranes as already described above.

For instance, the composition or the extract can, by virtue of their barrier effect, facilitate the repair of damaged skin or mucosal tissue, with entailed restoration of a healthy and elastic skin or mucous membrane.

The skin lesions according to the present invention may be, in case no cicatrizing and/or antimicrobial active principles be associated with the fractions of the invention, lesions that may involve also tissue underlying the skin and in which no open wounds are present.

By skin lesions not involving the presence of open wounds, according to the present description, are meant those lesions in which the superficial layer of the skin, and the underlying layers, though not being wounded, are particularly fragile, irritated and damaged.

Non-limiting examples of this type of lesions are represented by first-degree burns, first-degree decubitus lesions, pressure lesions, newly-cicatrized rashes, wounds or burns, irritations, erhytemas.

In association with active principles suitable for the treatment of skin lesions with open wounds, the fractions of the invention could be as adjuvants of said treatment thanks to their barrier effect.

The compositions or the fractions (or mixtures thereof) of the invention could then be used for the treatment or the prevention of skin lesions not involving the presence of open wounds or in the prevention or slowing down of worsenings of the same or, in association with suitable active principles not extracted from liquorice, in the treatment of skin lesions with open wounds.

The compositions according to the invention could advantageously comprise further components, for instance of plant origin, having, e.g., emollient, digestive, prokinetics, cholagogue, carminative, prebiotic, relaxing, excipient, preservative, humectant properties, besides active principles of natural origin not extracted (derived) from liquorice.

When the composition or one or more of the fractions of the invention are used for the protection of the skin, the application thereof will be topical. The embodiments of the compositions for topical use are reported hereinafter in the description.

The composition according to the invention could be made, e.g., as oil-in-water emulsion, water-in-oil emulsion, oil-in-gel emulsion or gel-in-oil emulsion, multiple emulsions, sprays, and anhydrous formulations (pomade, gel, paste, cream, ointment) and will comprise one or more excipients suitable for the making of the desired end form.

Such excipients could be, e.g., emulsifying agents (cetearyl alcohol, cetearyl glucoside, hydrogenated castor oil), rheological additives, antioxidants (like, e.g., vitamins, tocopherols or other antioxidants known in the field).

The emulsifying agent could be a surfactant, which by lowering the interfacial tension decreases the free energy of the system; alternatively, also non-surfactant substances can be used, such as gum arabic, gelatine, hydrophilic colloids or finely subdivided powders (e.g., talc). In one embodiment, the excipients could be present at an overall concentration in weight comprised between 3 and 8%, such concentration being of course indicative, taking in account that anyhow the technician in the field will know how to adapt the concentration of the necessary excipients according to the embodiment he/she intends to prepare without additional inventive activity.

Moreover, the composition could comprise perfuming and/or colouring agents making its odour pleasant, like, e.g., one or more essential oils like, e.g., lavender essential oil, melaleuca (tea tree), lemon, mint, orange essential oil, and/or colouring agents enabling, e.g., to easily recognize the zones of application of the composition, the colouring being, e.g., temporary, so as not to interfere with other, subsequent applications of the composition.

In one embodiment, said colouring and/or perfuming agents are comprised at an overall concentration in weight between 0.001 and 3%.

By "overall concentration in weight" it is meant the concentration in weight in the composition of the sum of the various excipients, or the concentration in weight in the composition of the sum of the various perfuming and/or colouring agents present in the composition.

As to the use of the composition in the protection of the skin, the invention also relates to medical devices like, e.g., medicated plasters, medicated gauzes, medicated bandages, medicated tissues, medicated tampons, medicated diapers (pads), i.e. plasters, gauzes, bandages, tissues, tampons or diapers (pads) which comprise, or be at least partly covered by, or be at least partly soaked with the described composition of the invention in the most appropriate form.

The gauzes could be made as fatty gauzes, and so the bandages and the plasters, by using the composition made in the form, e.g., of ointment, paste or cream. The tissues could be soaked with the composition in the form of emulsion of oil with water or gel, and the diapers (pads) or the tampons could be made by inserting the composition in the suitable layers known in the field for the insertion of protective or anti-irritating compositions.

Such products, like, e.g., diapers, tampons (commonly called "sanitary napkins") for women and pads for adult incontinence, are commonly used, e.g. in the babyhood/childhood-related field, in the feminine field and in the geriatric field, and the technician in the field will know where to insert the composition described herein, and which embodiment thereof be the most suitable one, with no need of specific teachings and by basing him/herself solely on techniques conventional in the field.

Such devices will be applicable on the part to be treated and/or protected by way of prevention.

As already indicated above, the composition of the invention can be a composition for topical use that could be made in the form of oil-in-water emulsion, water-in-oil emulsion, multiple emulsions, sprays, and anhydrous formulations (pomade, ointment, gel, paste, cream, spray) according to techniques commonly used in the field. The formulation indicated herein as "spray" could be an anhydrous formulation or even an emulsion formulation, the form with an atomizer enabling also self-applications in zones harder to reach (like, e.g., the back), useful in all those cases in which the formulation is used, e.g., to alleviate sun rashes, erythemas, or skin irritations of various kind.

According to another embodiment, one or more of the fractions of the invention or the composition of the invention could be used, thanks to their mucoadhesive and barrier effect, for the protection of mucous membranes.

In this case as well, such protection could be a preventive protection, e.g. in all those cases envisaging an administration of drugs having the side effect of attacking the mucous membranes, or in those patients exhibiting conditions of recurring irritation of the same. In other cases, the protection could be instead a protection for curative purposes or in order to avoid the worsening of irritated or partially compromised mucous membranes.

In these cases, the administration of one or more of the fractions A, B, C of the composition could be topical for all those mucous membranes on which a topical administration is possible (e.g., buccal, rectal, vaginal, nasal mucosa) or oral in the cases in which said membranes be, e.g., intestinal or gastric mucous membranes.

The compositions for the protection of the mucous membranes could therefore be made in the form of capsule, tablet, pill, granules, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

In case of formulations for topical use, the above indications related to the compositions for the protection of the skin could be followed, or syrups, rinses, sprays could be made, e.g. for the protection of the mucous membranes of the mouth, throat, nose.

For application on the rectal mucosa, suppositories or enemas or microenemas could be used, with the excipients known to the technician in the field for the making of such compositions.

As already mentioned, the composition of the invention could be in the form of capsule, tablet, pill, hard gelatine, soft gelatine, granules, powder, syrup, elixir, suspension, emulsion. For oral administration the composition could be made in the form of daily unit dosages or of fractions of daily unit dosages (e.g., 2, 3, 4, 5, 6, or more capsules, tablets, lozenges, granule or powder single-doses, or gelatins could be taken over the day, in accordance with the judgment of the treating doctor), and may contain conventional excipients, including, e.g., binding agents, like gum arabic, gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, like potato starch; and wetting agents like sodium laurylsulphate. The tablets can be coated according to methods well-known in the standard pharmaceutical practice.

The composition could also be made in a liquid or semi-liquid form, as a suspension, emulsion, solution for oral administration, and could optionally contain natural aromatizing agents giving a palatable taste thereto.

The composition in the form of powder or granule could be pre-metered in suitable containers and ready for use, either by ingestion as such or to be resuspended in an appropriate liquid such as water, tea, etc. In this case as well, the composition could contain natural aromatizing agents giving a palatable taste thereto.

Evidently, all of the above-indicated excipients could be used in a pharmaceutically acceptable grade.

In one embodiment, the composition as described herein, in any one of the above-indicated embodiments, could be in the form of pharmaceutical composition, i.e. comprise pharmaceutical-grade ingredients, or it could be or be introduced into a medical food or into a medical device.

The composition according to the present description could be made in the form of pharmaceutical composition or of medical device according to any one of the classes described in Directive 93/42/EEC on medical devices (comprising also substances and not only "devices" in the mechanical sense of the term), or in the form of medical food, food supplement, or in any form according to the regulatory provisions of the Country in which said composition will be produced.

The medical device or the medical food could also contain as ingredients other components, comprising e.g. combinations of vitamins, mineral salts and other substances directed at diet supplementing.

One or more of the fractions of the invention or the composition of the invention will therefore be useful for their barrier effect properties and, when present, mucoadhesion properties, in all those cases in which the protection of skin or mucous membranes is needed or desirable; those may be cases in which a drug that attacks mucous membranes or skin has to be administered, therefore in order to prevent or limit the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes sustain irritations or alterations, therefore a barrier effect can prevent or limit damages on the skin or the mucous membrane.

The invention also relates to a process for the treatment or for the prevention of the onset or the worsening of skin lesions not involving the presence of open wounds or for the treatment of open wounds, wherein such process comprises one or more applications of the composition of the invention or of the medical device comprising it once or more per day on the concerned part.

The application of the composition, for instance, could be repeated whenever needed (e.g. at each change of pad in case of prevention of incontinence-related rashes) or once, twice, thrice, four or more times per day in general.

The invention also relates to a process for the preparation of a composition according to any one of the embodiments described above, wherein fractions of liquorice extract depleted in glycyrrhizic acid as described herein (fractions A, B, C) or mixtures thereof are mixed with at least one among excipients and/or substances of natural and/or plant origin (glycyrrhizic acid excluded) having emollient, digestive, prokinetics, cholagogue, carminative, prebiotic, relaxing, excipient, preservative, humectant properties as described above.

Among these, e.g., could be used one or more of: gentian root extract, boldo leaves extract, milk thistle fruits extract, artichoke leaves extract, taraxacum root extract, anise fruit extract, rosemary leaves extract, mint leaves extract, marjoram leaves extract, cumin fruit extract, coriander fruit extract, ginger root extract, fennel fruit extract, caraway fruit extract, chamomile flowers, althea root extract, linseed extract, barley seed extract, aloe leaves gel, charcoal, inulin.

Object of the present invention is also a method for the protective (preventive or curative) treatment of the skin or mucous membranes providing the administration of one or more of the fractions of the invention or the composition of the present invention to a patient in need thereof. Such administration could also be concomitantly with the administration of other drugs.

The absence of pharmacologically active principles of liquorice in the fractions or in the composition makes such products particularly suitable to administration concomitantly with other drugs, as a side effect of interaction between the extract or the composition of the invention and the drug co-administered or concomitantly administered is markedly unlikely.

Therefore, by "protective treatment" for the purposes of present invention, it is meant a treatment of damaged, irritated or irritable (e.g., if the use of an irritating drug is envisaged) skin or mucous membranes, wherein one or more fractions or the composition of the invention form a protective film thanks to the mucoadhesive power of the fraction of the invention, and thereby enable a healing of the damaged, wounded and/or irritated mucous membranes, or prevent damage thereto which may be caused by therapeutic treatments that must be administered to the patient, by administering the fraction or composition of the invention before the drug.

Alternatively, one or more fractions (A, B and/or C) or the composition could be administered together with active principles that is desirable to retain longer on the mucous membrane in order to prolong or make more effective the therapeutic effect thereof.

A non-limiting example of the method of treatment and/or of prevention of the skin or mucous membranes could comprise the administration of a daily dosage, subdivided into a single dose or plural doses, described of the mixture or the composition according to the present description, for a period of time of between one and six weeks, e.g. of between three and six weeks or even for a period of time higher than six weeks, in accordance with the judgment of the treating doctor.

Such administration could precede the administration of the drug even for a prolonged period, so as to optimize the health state of the skin or of the mucous membrane to be treated.

The treating doctor will know how to establish both the most appropriate dosage and the administration times also on the basis of the patient's health state, weight, sex and age.

One of the substantial differences between the structure of the skin and that of the mucous membranes is represented by the absence, in the mucous membranes, of a selective barrier such as the corneous layer. Oral mucous membranes contact with noxious or irritating substances present in the environment (pollutants, pathogenic microorganisms, etc.) can therefore cause a high penetration of said substances both inside the mucous membranes and in the related airways (bronchi, lungs, etc.) causing inflammatory and/or allergic pathologies.

The protective effect of the extract of the present invention was assessed by mucoadhesion assays and by barrier effect assay.

The former aim at evaluating the mucoadhesivity of the product under examination in order to establish whether such product has the ability to adhere to mucous membranes and consequently exert a protective action.

For instance, a simple model for evaluating mucoadhesivity of the product of interest is available.

This model evaluates the mucoadhesive ability of the sample by measuring its ability to bind to mucin disposed on an inclined plane, allowing the moving away by sliding of the sample lacking mucoadhesive ability.

For evaluation of the barrier effect an *in vitro* model is available enabling to evaluate the hindrance of the sample to transit of LPS, accountable, in the model, for the freeing of mediators such as interleukin 6 (IL-6) measured semi-quantitatively by ELISA assay.

### Barrier effect assay

The barrier effect assay is a biological-type *in vitro* assay employed for assessing the ability of finished products and/or raw materials to protect, through the formation of a thin "insulating" layer, mucous membranes and skin from contact with environmental contaminants (dust, pollens, microorganisms, etc. )

The assay was developed to simulate *in vitro* the action exerted by products that are applied on skin and/or mucous membranes with the aim of creating a protective film against external aggressors.

The model takes advantage of the principle whereby cells subjected to contact with an inflammatory agent produce and secrete pro-inflammatory mediators (cytokines) in the extracellular environment in an amount related to the degree of inflammation caused; within a certain range, a direct proportionality exists between concentration and times of exposure to the inflammatory agent and amounts of cytokines released.

The experimental model adopted provides two chambers physically separated by a semi-permeable membrane (0.4µm pores). Cells are seeded in the lower (bottom) chamber whereas the upper chamber accommodates the inflammatory agent; on the semi-permeable membrane separating the two chambers a thin film of the sample under analysis is stratified to highlight, if present, a barrier effect to the free transit of the inflammatory agent.

The semi-permeable membrane allows passage of the inflammatory agent in the lower chamber and constitutes the support on which the sample to be assayed is stratified. Depending on the "insulating" ability of the sample, a decrease of LPS migration from the upper chamber to the bottom one will be had (therefore a lesser stimulation of cells to cytokine production).

EXAMPLES

**Example 1**

**Preparation of fractions A B and C**

Liquorice roots were fragmented and subjected to two steps of extraction in ethanol at decreasing concentrations, performing a step in 96% ethanol and a step in 5% ethanol.

The extraction temperature is of about 40°C, in case of alcoholic extraction, and of about 50°C, in case of aqueous extraction.

The alcoholic extracts obtained as described were gathered in a ratio of about 50:50, in any case keeping the alcoholic grade of the resulting mixture between 40° and 60°, preferably between 45° and 55° alcoholic grades. The resulting mixture was subjected to centrifugation for a time of 1-5 minutes at a rotation rate equal to 3,500 rpm; the precipitate constituting fraction A was recovered. The supernatant was concentrated by ethanol evaporation with use of a thin film distillation system according to the standard protocol, providing the feeding of the extract to be concentrated at a rate of about 500 l/h; (operation was by setting a residual vacuum of 0.6-0.8bar and the fluid heating the evaporation walls was set at 140°C) thereby obtaining, after ethanol elimination, a concentrated aqueous fraction and a second precipitate (fraction D) characterised by a glycyrrhizic acid concentration of $\leq 0.8\%$ in weight. The concentration process is conducted by concentrating the volumes of a factor of from 3:1 to 10:1.

The aqueous extract so obtained was filtered on a panel filter with a cutoff of 25 micrometers.

The filtrate so obtained was subjected to one or more steps of ultrafiltration on a membrane with a cutoff of 2,500 daltons and/or on two membranes in combination, of 10,000 and 2500 daltons.

The permeate (Fraction B) thus obtained comprised glycyrrhizic acid at a concentration in weight not greater than 0.05%.

The concentrated aqueous extract after settling and recovery of the clarified supernatant, or after filtration and recovery of the filtrate, was subjected, alternatively to the above-described ultrafiltration process, to a treatment with a high-porosity adsorbing resin, in particular a styrene and DVB copolymer resin of ADS TQ 318 type was used; evidently, any adsorbing resin with similar features could be used in the carrying out of the present invention.

The remaining active principles are retained by the resin and the eluate is collected.

By this process a fraction (fraction C) comprising glycyrrhizic acid at a concentration in weight not greater than 0.4% is obtained.

In this case, a standard process for obtainment of the extract by treatment on resin entails the introduction of the concentrated aqueous extract in a chromatographic column containing the adsorption resin. The feed fluid must have suspended solids indicatively comprised between 0.2 at 1° Bix, the feed range is between 1 and 4BV/hour and preferably 2 BV/hour. The corresponding aqueous eluate is collected and subjected to drying by means of lyophilisation or desiccation. The substances adsorbed into the resin are eluated using 96% ethyl alcohol or methanol or acetone, preferably 96% ethyl alcohol. In this latter case, the alcohol eluate is subjected to desiccation or to lyophilisation (after having added in this latter case water at a ratio of 1:1 v/v with alcohol eluate and having evaporated the ethyl alcohol), useful for other purposes.

**Example 2**

**Evaluation of adhesion percentage by inclined plane with mucin.**

Measurements are performed by an equipment comprised of a 45°-inclined plane, thermostated to 37°C; below said plane a microbalance is set, which performs measurements at regular 1-s intervals and prints recorded measurements on paper. Performed measurements are then transcribed and reprocessed on Excel spreadsheet.

The inclined plane acts as support for the biological substrate, consisting of a mucin film set up by depositing, on a plexiglas plane kept horizontal, a volume equal to 3ml of a suspension of porcine gastric mucin at an 8% concentration (w/w) in a phosphate buffer, pH 6.4.

The dispersion is then brought to dryness (optionally also at the temperature of 44°C) so as to obtain a film of known surface area equal to 33.6 cm$^2$.

An exactly weighed amount of the fractions according to the invention is dissolved in a solvent. The fraction is then deposited on the measurement plane with a multichannel pipette at constant time and rate during 6 seconds.

The multichannel pipette is calibrated so as to meter the solution (or dispersion) over the work plane. The total amount is measured at each assay (the weight of said volume is measured before the assay and depends on the density of the sample under analysis).

The sample, which will fall on the microbalance at the end of the run, is loaded in the top part of the inclined plane, containing the mucin film, and let slide thereon. The amount of fallen sample is measured by recording the weight variation as a function of time. The assay as made herein lasts 40 seconds, though usually after 20 seconds there is no weighing variation.

Measurements in the absence of the mucin film on the inclined plane are also carried out (measurements indicated as blank) with the same amount of sample and under the same assaying conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

$$\% \text{ difference} = (\text{adhered mucin } \% - \text{adhered blank } \%)/\text{adhered } \% \text{ of the blank}$$

Where:

- adhered mucin %= sample percentage remained adhered to the mucin film at the end of the measurement
- adhered blank % = sample percentage at the inclined plane at the end of the blank measurements.

### Example 3.

### Barrier effect assay carried out for each of the fractions A, B and C and on the reference extract

(For the barrier effect assay, two chambers physically separated by a semi-permeable membrane (0.4$\mu$m pores) were used. Human fibroblast cells were seeded in the lower chamber, whereas the inflammatory agent LPS (purified E. Coli lipopolysaccharide) was introduced into the upper chamber; on the semi-permeable membrane separating the two chambers a thin film of fractions of liquorice extract as described in the present invention was stratified.

Induced inflammatory response was estimated through semi-quantitative dosage of Interleukin 6 (IL6) cytokine released in the culture medium of the lower chamber: barrier effect assessment was obtained by comparison with the positive control in which the two chambers were separated by the same type of semi-permeable membrane, free however from any barrier.

The greater the barrier effect exerted by the stratified film of substance, the lesser the inflammatory action observed in the cells present in the chamber therebelow, as the inflammatory agent in the chamber thereabove will have greater difficulty to cross the semi-permeable chamber.

As to the threshold value above which it may be said that a substance causes a barrier effect in the assay reported herein, a value equal to 15% of inhibition as compared to the control was identified by the Inventors, during the setting up of the assay, on the basis of assays carried out on substances known to have a barrier effect.

Then the barrier effect of the fractions as described herein was evaluated.

The data reported below show how the fractions of the extract according to the description have a remarkable barrier effect, compared to the reference extract.

The assessment assay was carried out as follows through a method developed to simulate *in vitro* the protective action of substances and formulations which, when applied to the skin and mucous membranes *in vivo,* form an "insulating" film against environmental agents.

The model takes advantage of the principle whereby cells subjected to contact with an inflammatory agent produce and secrete pro-inflammatory mediators (cytokines) in the extracellular environment in an amount related to the degree of inflammation caused. The greater the amount of inflammatory agent reaching the cells, the greater the amount of cytokines released.

The model envisages the arrangement of two chambers physically separated by a semi-permeable membrane which allows the passage of sufficiently small-sized solutes.

In the lower chamber, consisting of a well containing plates for cell cultures, HuDe cells (no. BS PRC 41, purchased from the Istituto Zooprofilattico di Brescia, Italy) are grown, while the upper chamber, consisting of an insert for complex cell cultures (transwells), accommodates the inflammatory agent.

On the surface of the semi-permeable membrane of the insert separating the two chambers, before the introduction of the inflammatory agent in the upper chamber, a thin film of the sample being examined is stratified to assess any BE upon the free passage of the inflammatory agent.

As a function of the insulating capabilities of the sample, there will be had a decrease of the migration of the inflammatory agent from the upper chamber and, as a consequence, a lesser stimulation of the cells to produce cytokines. The extent of the inflammatory reaction is estimated through the semi-quantitative dosage of cytokines released in the culture medium of the lower chamber, in particular of interleukin 6 (IL-6).

As a control a similar experiment is used in which no sample is stratified on the membrane, thus making it possible to measure the effect of the inflammatory agent without any barrier in addition to the semi-permeable membrane.

Further, an internal control is used in which the cultured cells are pre-treated with the substance adapted to induce the release of the marker and the sample is placed on the semi-permeable membrane in the absence of said substance, one or more measurements in time are thus performed of the quantity of marker in the culture medium of said internal control. In the internal control, the cells are therefore stimulated first with the inducing substance and then there has to be assessed whether the sample which may pass the membrane and go into the cells pushed by the medium above has any effect in decreasing the release of the marker not related to the barrier effect. For instance, when an inflammatory agent is used as the inducing substance, the internal control makes it possible to understand if the reduction in the concentration of cytokines in the culture medium is due to the barrier effect or if the sample that may pass into the cells pushed by the medium above has any effect in reducing the inflammatory response independently of the barrier effect.

The barrier effect (BE) is expressed as a percentage of the reduction of the release of IL-6 and is calculated through comparison with the positive control in which the two chambers are separated by the same type of semi-permeable membrane without the barrier created by the sample.

### 3.1 PREPARATION OF THE CELL CULTURE:

For each assayed sample, HuDe line cells were seeded in the wells of a cell culture plate, one for the barrier assay (BA) and another one for the internal control at a density of 40,000 cells /ml in MEM medium supplemented with 10% bovine serum (FBS); 1 ml of cell suspension per well.

The cells were treated with the SAMPLE (CAM), with the POSITIVE CONTROL (C+) (inflammatory agent without sample), and with the NEGATIVE CONTROL (C-) (medium only) and each assay was carried out in triplicate.

The plates were incubated at 37°C, overnight (22-24 hours).

### 3.2 PREPARATION OF INSERTS FOR COMPLEX CELL CULTURES

Cell Culture Inserts (Becton Dickinson) for complex cell cultures were placed on other plates, and on each of them a fixed amount of collagen of 0.1 mg/ml was supplied. The plates were incubated at 37°C, overnight (22-24 hours).

### DAY 2

### 3.3 VERIFICATION OF STATE AND LEVEL OF CELL CONFLUENCY

In order to proceed with the experiment, a confluency not lower than 95% is required.

### 3.4 COLLAGEN LAYER DRYING

From the two plates (BA and IC) with the inserts the collagen was removed and the insert left under the flow of the hood for the time required to let them dry completely (10÷15 minutes).

3.5 BARRIER ASSAYS (BA)

The steps described below were carried out in the culture plate for the BA.

Setting up of the sample layer in the BA:

On the sample's semi-permeable membrane 100μl of a composition based on each of the fractions of liquorice extract (2%) (the assay was performed for each fraction) described in the invention were inoculated and allowed to stratify for 20 minutes while in the C+ and C- inserts nothing was added. Once the 20 minutes had elapsed, excess sample was eliminated and the membranes washed with PBS according to procedures specified by the protocol.

Addition of LPS (inflammatory agent) to BA inserts

Once the sample layer had dried, in the first three CAM inserts and in the three C+ ones, 300μl of the LPS (membrane lipopolysaccharide) solution were inoculated at the concentration of 1 μg/ml while in the remaining three of the C- 300 μl of MEM medium with 5% FBS were added. The inserts were inserted in their respective wells with the cells and the plates were incubated for 1 h at 37°C and under an atmosphere enriched with 5% $CO_2$ overnight (22-24 hours).
Once completed the 1 h incubation, the inserts were removed and discarded and the plates were incubated again overnight (22-24 hours).

3.6 INTERNAL CONTROL (IC) ASSAY:

The internal control assay was carried out concomitantly with the BA.

Exposure of IC cells to LPS:

Once dried, in the first six inserts of IC, three for the sample to be analysed, CAM, and three for the C+, 300μl of the LPS solution were inoculated, while in the remaining three of the C- 300μl of the medium were added.
The inserts with LPS and MEM were then inserted in the wells with IC cells and all incubated for 1 h.

LPS removal and IC membrane drying:

Once completed the 1 h incubation, the inserts were removed from the wells with the cells and transferred to the empty plate, while the plate with the cells was placed in an incubator.
The LPS solution still present was removed from the inserts, the latter were subjected to a rapid wash with ultrapure sterile water and allowed to dry.

Arrangement of sample layer in the IC:

On the semi-permeable membrane of the three inserts for the sample, 100μl of a composition based on each of the fractions of liquorice extract (2%) according to the invention were inoculated and allowed to stratify for 20 minutes while in the C+ and C- inserts nothing was added. Once the 20 minutes had elapsed, the excess sample was eliminated and the membranes were washed with PBS according to procedures specified by the protocol.

Addition of LPS to IC inserts

Once the inserts with the sample were ready, 300 μl of medium were added to all inserts (CAM, C+, C-). The inserts were inserted in their respective wells with the cells and the plates incubated for 1 h at 37°C.
Once completed the 1 h incubation, the inserts were removed and discarded and the plates incubated again overnight (22-24 hours).

DAY 3

4.7 SUPERNATANT COLLECTION AND ENZYME IMMUNOASSAY

Once the 22-24 hours had elapsed, the supernatants were collected from the BA and the IC plates for performing the ELISA assay and the semi-quantitative dosage of IL-6.

BARRIER EFFECT (BE) ASSESSMENT

The BE of a substance or compound is expressed as *% reduction in the release of IL-6 cytokine* by cells exposed to LPS wherein the sample has been assayed relative to the positive control (C+) wherein the cells have only been exposed to LPS.

$$BE = \text{\textit{\% reduction in release of IL-6 cytokine}} = 100 - [\text{ (pg/µL cytokines released from sample / pg/µL cytokines released from C +) x 100]}$$

Table 1 reported above shows that each of the assayed fractions has a greater barrier effect compared to the non-fractionated reference extract, and in fractions A and C also a maintaining, or even a maintaining of mucoadhesive abilities compared to the non-fractionated reference extract.

The data obtained show that the fractions of the invention are suitable for the uses described and claimed.

**Example 4**

**QUANTITATIVE HPLC DETERMINATION OF GLYCYRRHIZIC ACID IN HPLC**

Preparation of analysis solution.

Accurately weigh 0.50g $\pm$0.025g of ground sample. Extract sample for 30min with 100ml of 25% ammoniacal solution by ultrasounds at the temperature of 35°C $\pm$3°C. Centrifuge and decant the extract in a 100ml volumetric flask. Filter with a 0.45$\mu$m cellulose acetate filter and inject in HPLC system.

Chromatographic conditions:

Chromatographic column: C18, 100x4.6mm 5$\mu$ + C18 pre-column, 5$\mu$, 4x3mm
column temperature: 20°C
detector: UV-Vis $\lambda$= 254nm
flow: 1.5ml/min
injection volume: 10ul

The mobile phase consists of:

A = $CH_3COOH$/ultrapure $H_2O$/$CH_3CN$ (6/64/30) (v/v/v)
B = $CH_3CN$
Elution: under isocratic conditions for 19 minutes

Standard ammoniacal glycyrrhizin is solubilised in 25% methanol.

**Claims**

1.  A fraction of liquorice root and/or taproot extract depleted in glycyrrhizic acid, wherein said glycyrrhizic acid has a concentration expressed as weight percent of ≤0.8 and a ratio between glycyrrhizic acid and water-soluble macromolecules with a molecular weight of >25000 Da of ≤1:20.

2.  The fraction according to claim 1, wherein said glycyrrhizic acid has a concentration expressed as weight percent of ≤0.4% and a ratio between glycyrrhizic acid and water-soluble macromolecules with a molecular weight of 25000 Da of ≤1:50.

3.  The fraction according to claim 1, wherein said glycyrrhizic acid has a concentration expressed as weight percent of ≤0.05% and a ratio between glycyrrhizic acid and water-soluble macromolecules with a molecular weight of >25000 Da of ≤1:500.

4.  The fractions according to any one of claims 1 to 3 or mixtures thereof for use in a treatment for the protection or skin or mucous membrane.

5. The fractions according to claim 4, wherein said mucous membrane is the oral mucosa, gastric mucosa, intestinal mucosa, the nasal mucosa, the vaginal mucosa, the uterine mucosa, the rectal mucosa.

6. A process for the preparation of a fraction of liquorice root and/or taproot extract depleted in glycyrrhizic acid comprising the following step:

   a. preparing a hydroalcoholic extract of liquorice root and/or taproot and subjecting said extract to centrifugation and/or decantation, obtaining a precipitated fraction A **characterized by** a concentration, expressed as weight percent, of ≤0.8 and a ratio between glycyrrhizic acid and water-soluble macromolecules with a molecular weight of >25000 Da of ≤1:20, and a clarified alcoholic extract, and said precipitated fraction A is collected.

7. The process according to claim 6, wherein said hydroalcoholic extract at step a. is prepared by subjecting liquorice root and/or taproot, optionally fragmented, to two or more extraction steps in decreasing concentrations of ethanol.

8. The process according to claims 6 or 7 wherein said two or more steps in a. comprise a step in 96% ethanol, and a step in 15%-5% ethanol.

9. The process according to any one of claims 6 to 8, further comprising the following steps:

   b. obtaining from the clarified hydroalcoholic extract prepared in a. a concentrated aqueous extract,
   c. decanting and/or centrifuging and filtering said aqueous extract, obtaining a water-soluble fraction,
   d. subjecting the water-soluble fraction to membrane ultrafiltration and collecting a fraction B, corresponding to the permeate obtained from said ultrafiltration, **characterised by** a glycyrrhizic acid concentration expressed as weight percent of ≤0.05% and a ratio between glycyrrhizic acid and water-soluble macromolecules with a molecular weight of >25000 Da of ≤1:500.

10. The process according to any one of claims 6 to 8, further comprising the following steps:

   b. obtaining from the clarified alcoholic extract prepared in a. a concentrated aqueous extract,
   c. decanting and/or centrifuging and filtering said aqueous extract, collecting a water-soluble fraction:
   d'. subjecting the water-soluble fraction to a step on adsorbing resin collecting a fraction C, corresponding to the eluate obtained by said step on resin, **characterised by** a glycyrrhizic acid concentration expressed as weight percent of ≤0.4% and a ratio between glycyrrhizic acid and water-soluble macromolecules with a molecular weight of >25000 Da of ≤1:50.

11. A pharmaceutical carrier consisting in one or more fractions according to any one of claims 1 to 4, wherein said carrier promotes the adhesion of active principles to the mucous membrane.

12. A composition comprising one or more fractions of a liquorice extract according to any one of claims 1 to 4.

13. The composition according to claim 12 for use in a treatment for the protection of skin or mucous membrane.

14. The composition according to claim 13 wherein said mucous membrane is the oral mucosa, gastric mucosa, intestinal mucosa, the nasal mucosa, the vaginal mucosa, the uterine mucosa, the rectal mucosa.

15. The composition according to any one of claims 12 to 14 wherein said composition is or is comprised in a pharmaceutical composition, is comprised in or consists in a medical device, or is comprised in a medical food.

Fig. 1a

c. clarified aqueous extract

or d'.

Adsorbing resin
stirene DVB copolymer

Retentate

d.
1-ULTRAFILTRATION
MWCO 2500
DALTON

Retentate > 2500
Dalton,

ELUATE
FRACTION C

PERMEATE
FRACTION B

Fig. 1b

Europäisches
Patentamt

European
Patent Office

Office européen
des brevets

# EUROPEAN SEARCH REPORT

**Application Number**

EP 14 18 7826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WITTSCHIER N ET AL: "Aqueous extracts and polysaccharides from Liquorice roots (Glycyrrhiza glabra L.) inhibit adhesion of Helicobacter pylori to human gastric mucosa", JOURNAL OF ETHNOPHARMACOLOGY, ELSEVIER IRELAND LTD, IE, vol. 125, no. 2, 7 September 2009 (2009-09-07), pages 218-223, XP026498475, ISSN: 0378-8741, DOI: 10.1016/J.JEP.2009.07.009 [retrieved on 2009-07-14] * the whole document * * see esp. p. 219, right column, 2nd and 3rd paragraph * ----- | 1-15 | INV. A61P17/16 A61K36/484 |
| X | WO 97/00264 A1 (EXTRAITS VEGETAUX ET DERIVES E [FR]; BOZZI ROGER [FR]; GAVACH CLAUDE []) 3 January 1997 (1997-01-03) * the whole document * ----- | 1-10 | |
| X | VAN MARLE J ET AL: "Deglycyrrhizinised liquorice (DGL) and the renewal of rat stomach epithelium", EUROPEAN JOURNAL OF PHARMACOLOGY, ELSEVIER SCIENCE, NL, vol. 72, no. 2-3, 19 June 1981 (1981-06-19), pages 219-225, XP023839768, ISSN: 0014-2999, DOI: 10.1016/0014-2999(81)90276-4 [retrieved on 1981-06-19] | 1-5, 12-15 | **TECHNICAL FIELDS SEARCHED (IPC)** A61K |
| Y | * the whole document * ----- -/-- | 6-11 | |

The present search report has been drawn up for all claims

| Place of search | Date of completion of the search | Examiner |
|---|---|---|
| Munich | 28 January 2015 | Greif, Gabriela |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

Application Number

EP 14 18 7826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BROGDEN R N ET AL: "Deglycyrrhizinised liquorice: a report of its pharmacological properties and therapeutic efficacy in peptic ulcer", DRUGS, ADIS INTERNATIONAL LTD, NZ, vol. 8, no. 5, 1 January 1974 (1974-01-01), pages 330-339, XP009178300, ISSN: 0012-6667 | 1-5, 12-15 | |
| Y | * the whole document * | 6-11 | |
| X | MORGAN R J ET AL: "The protective effect of deglycyrrhinized liquorice against aspirin and aspirin plus bile acid-induced gastric mucosal damage, and its influence on aspirin absorption in rats", JOURNAL OF PHARMACY AND PHARMACOLOGY, JOHN WILEY & SONS LTD, LONDON; GB, vol. 35, no. 9, 1 September 1983 (1983-09-01), pages 605-607, XP009178294, ISSN: 0022-3573 | 1-5, 12-15 | |
| Y | * the whole document * | 6-11 | TECHNICAL FIELDS SEARCHED (IPC) |
| X | MORGAN R J ET AL: "STUDIES ON THE PROTECTIVE EFFECT OF DEGLYCYRRHINIZED LICORICE AGAINST ASPIRIN AND ASPIRIN PLUS BILE ACID INDUCED GASTRIC LESIONS IN RATS", CLINICAL SCIENCE, BIOCHEMICAL SOCIETY AND THE MEDICAL RESEARCH SOCIETY, LONDON, GB, vol. 63, no. 3, 1 January 1982 (1982-01-01), page 19p, XP009178295, ISSN: 0143-5221 | 1-5, 12-15 | |
| Y | * the whole document * | 6-11 | |

-/--

The present search report has been drawn up for all claims

| Place of search | Date of completion of the search | Examiner |
|---|---|---|
| Munich | 28 January 2015 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches
Patentamt

European
Patent Office

Office européen
des brevets

**EUROPEAN SEARCH REPORT**

Application Number

EP 14 18 7826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ISBRUCKER R A ET AL: "Risk and safety assessment on the consumption of Licorice root (Glycyrrhiza sp.), its extract and powder as a food ingredient, with emphasis on the pharmacology and toxicology of glycyrrhizin", REGULATORY TOXICOLOGY AND PHARMACOLOGY, ACADEMIC PRESS,NEW YORK, NY, US, vol. 46, no. 3, 1 December 2006 (2006-12-01), pages 167-192, XP024915298, ISSN: 0273-2300, DOI: 10.1016/J.YRTPH.2006.06.002 [retrieved on 2006-12-01] | 1-5, 12-15 | |
| Y | * page 174, right-hand column, paragraph 2 - page 175, right-hand column, paragraph 2 * | 6-11 | |
| | ----- | | |
| Y | DATABASE WPI Week 201202 Thomson Scientific, London, GB; AN 2011-Q38964 XP002726063, & CN 102 258 625 A (XINJIANG UIGHUR AUTONOMOUS REGION ANIMAL) 30 November 2011 (2011-11-30) * abstract * | 6-11 | TECHNICAL FIELDS SEARCHED (IPC) |
| | ----- | | |
| | -/-- | | |

The present search report has been drawn up for all claims

| Place of search | Date of completion of the search | Examiner |
|---|---|---|
| Munich | 28 January 2015 | Greif, Gabriela |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................
& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 14 18 7826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LUTOMSKI J: "CHEMIE UND THERAPEUTISCHE VERWENDUNG VON SUESSHOLZ", PHARMAZIE IN UNSERER ZEIT, VCH VERLAGSGESELLSCHAFT, WEINHEIM, DE, vol. 12, no. 2, 1 January 1983 (1983-01-01), pages 49-54, XP001026284, ISSN: 0048-3664 * the whole document * * but see especially p. 53, left column, 2nd paragraph, and middle column, 2nd paragraph. * | 1-15 | |
| Y | SMART ET AL: "The basics and underlying mechanisms of mucoadhesion", ADVANCED DRUG DELIVERY REVIEWS, ELSEVIER, AMSTERDAM, NL, vol. 57, no. 11, 3 November 2005 (2005-11-03), pages 1556-1568, XP027771403, ISSN: 0169-409X [retrieved on 2005-11-03] * the whole document * | 1-15 | TECHNICAL FIELDS SEARCHED (IPC) |
| A | CIRILLO G ET AL: "Molecularly imprinted polymers for the selective extraction of glycyrrhizic acid from liquorice roots", FOOD CHEMISTRY, ELSEVIER LTD, NL, vol. 125, no. 3, 1 April 2011 (2011-04-01), pages 1058-1063, XP027477871, ISSN: 0308-8146, DOI: 10.1016/J.FOODCHEM.2010.09.077 [retrieved on 2010-10-13] * the whole document * | 1-11 | |

The present search report has been drawn up for all claims

| Place of search | Date of completion of the search | Examiner |
|---|---|---|
| Munich | 28 January 2015 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                          EP 14 18 7826

This annex lists the patent family members relating to the patent documents cited in  the above-mentioned European search report.
The members are as contained in the European Patent Office EDP file on
The European Patent Office is in no way liable for these particulars which are merely given for the purpose of information.

28-01-2015

| Patent document cited in search report | | Publication date | Patent family member(s) | | Publication date |
|---|---|---|---|---|---|
| WO 9700264 | A1 | 03-01-1997 | FR | 2735477 A1 | 20-12-1996 |
|  |  |  | WO | 9700264 A1 | 03-01-1997 |
| CN 102258625 | A | 30-11-2011 | NONE | | |

EPO FORM P0459

**EP 2 857 068 A1**

**REFERENCES CITED IN THE DESCRIPTION**

**Non-patent literature cited in the description**

- **J.D. SMART.** *Advanced drug delivery reviews,* 2005, vol. 57, 1556-1568